# FASCICULE DE BREVET EUROPEEN

(11) **EP 3 914 847 B1**
(45) Date de publication et mention de la délivrance du brevet: **23.11.2022**
(21) Numéro de dépôt: 20700922.6
(22) Date de dépôt: 21.01.2020
(51) Int. Cl.: F16N 29/00, G01N 33/28

(54) **TÊTE MAGNÉTIQUE POUR DÉTECTEUR MAGNÉTIQUE DE PARTICULES MÉTALLIQUES ET DÉTECTEUR MAGNÉTIQUE POURVU D'UNE TELLE TÊTE**
MAGNETKOPF FÜR EINEN MAGNETDETEKTOR ZUM NACHWEIS VON METALLTEILCHEN UND MIT EINEM SOLCHEN KOPF VERSEHENER MAGNETISCHER DETEKTOR
MAGNETIC HEAD FOR A MAGNETIC DETECTOR FOR DETECTING METAL PARTICLES, AND MAGNETIC DETECTOR PROVIDED WITH SUCH A HEAD

(30) Priorité: 25.01.2019 FR 1900682
(43) Date de publication de la demande: 01.12.2021
(73) Titulaire: Safran Aerosystems Fluid, 77111 Soignolles en Brie (FR)
(72) Inventeur: COSOLETO, David, 77550 MOISSY-CRAMAYEL (FR); BOURBON, Patrick, 77550 MOISSY-CRAMAYEL (FR); SINDEZINGUE, Denis, 77550 MOISSY-CRAMAYEL (FR)
(74) Mandataire: Casalonga
(86) Numéro de dépôt international: PCT/EP2020/051425
(87) Numéro de publication internationale: WO 2020/152175

(56) Documents cités:
- EP-A1- 0 398 800
- FR-A1- 2 686 693
- JP-U- H0 564 751
- US-A1- 2014 347 032

## Description

La présente invention concerne les détecteurs magnétiques de particules métalliques qui sont susceptibles d'être présentes dans un circuit hydraulique.

L'invention concerne en particulier la détection magnétique de particules métalliques dans des moteurs ou dans des boîtes de transmission.

L'invention concerne plus particulièrement une tête magnétique destinée à être montée dans un détecteur magnétique, ainsi qu'un détecteur magnétique doté d'une telle tête magnétique.

Dans une application particulièrement intéressante de l'invention, le détecteur magnétique est destiné à assurer la détection de particules métalliques dans un circuit hydraulique d'un aéronef afin notamment par analyse de détecter des usures éventuelles de pièces mécaniques.

Dans l'état de la technique, les détecteurs magnétiques utilisent une tête magnétique qui comporte, d'une part, un ou plusieurs aimants, au moins deux électrodes en matériau électriquement conducteur isolées l'une de l'autre et placées à proximité de l'aimant de telle sorte que l'entrefer soit situé dans la zone de magnétisation de l'aimant et, d'autre part, des isolants qui assurent l'isolation électrique des électrodes. EP 0 398 800 A1 divulgue un tel détecteur magnétique comprenant une tête magnétique pour détecteur magnétique de particules métalliques dans un circuit hydraulique.

Les isolants sont couramment tubulaires et sont placés autour des aimants. Les électrodes sont reliées électriquement au calculateur de l'aéronef.

En présence de particules métalliques, celles-ci sont attirées par les aimants. Lorsqu'une quantité suffisante de particules se trouve dans la zone d'entrefer, la résistance électrique entre les électrodes diminue. Cette chute de résistance électrique est détectée par le calculateur de l'aéronef et est signalée au pilote de l'aéronef.

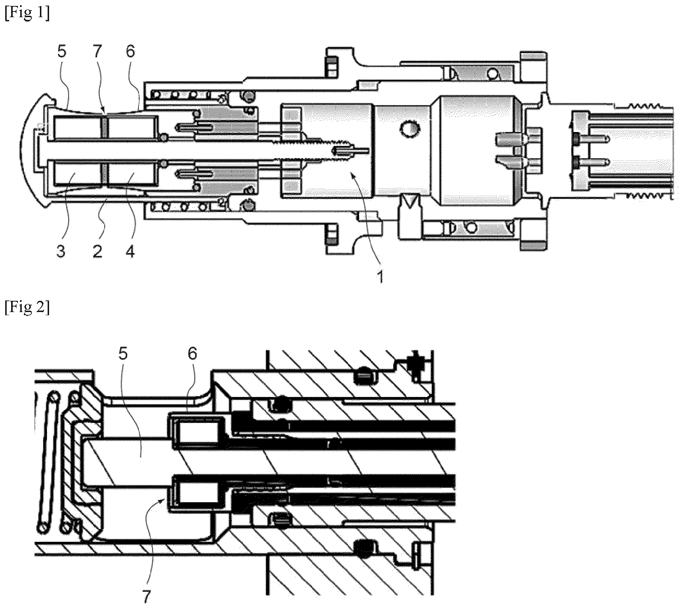

On a représenté sur la figure 1 un exemple de réalisation d'un détecteur magnétique de particules métalliques conventionnel.

Ce détecteur, qui est par exemple destiné à être monté dans un carter d'huile, comporte une tête magnétique 1 qui vient se monter dans un réceptacle 2 et qui comporte deux aimants 3 et 4 entourés par des électrodes 5 et 6 tubulaires respectives séparées par un entrefer 7.

Dans le mode de réalisation de la figure 1, l'entrefer 7 est un entrefer axial.

Comme le montre la figure 2, on connaît également des détecteurs magnétiques dont la tête est dotée d'un entrefer radial.

On voit en effet que la tête magnétique comporte ici un aimant annulaire. L'une des électrodes 5 est une électrode axiale, l'autre électrode 6 étant placée autour de l'aimant. L'entrefer s'étend ainsi radialement, en considérant l'axe général de la tête magnétique.

Il a été constaté que l'efficacité globale des détecteurs magnétiques de particules, communément appelés Bouchons Magnétiques Electriques (BME) ou désignés par les anglo-saxons par le terme de « Electrical Chip Detectors » (ECD) est assez faible.

Cette faible efficacité est due à un certain nombre de paramètres qui influent sur la captation et la détection des particules métalliques.

Il s'agit en particulier de la présence de zones d'écoulements turbulents, de flux d'huile s'écoulant hors de la portée magnétique des détecteurs,..., ou, de manière générale, des caractéristiques du système de lubrification.

Le faible taux de collecte des particules métalliques est également dû aux caractéristiques propres de l'aimant et au volume général de la tête magnétique qui limitent la zone de captation des particules.

Le but de l'invention et donc de pallier tout ou partie des inconvénients liés à l'utilisation des têtes magnétiques selon l'état de la technique et, en particulier, d'augmenter la puissance du champ magnétique créé par l'aimant, de manière à augmenter le taux de particules captées et la concentration des particules dans l'entrefer.

Il est donc proposé, selon un premier aspect, une tête magnétique pour détecteur magnétique de particules métalliques dans un circuit hydraulique, comprenant un corps axial comportant intérieurement au moins un aimant, au moins une première électrode délimitant une zone d'entrefer située dans le champ magnétique créé par l'aimant de sorte que l'aimant crée dans l'entrefer une zone d'alignement des particules, et des moyens de raccordement électrique des électrodes.

L'aimant est un aimant à aimantation diamétrale.

Cette aimantation diamétrale permet de présenter les zones de plus fort champ magnétique, à savoir les pôles, dans la zone de fluide comportant les particules à capter et, par conséquent, d'obtenir un taux de particules captées plus élevé.

Dans un mode de réalisation, la tête magnétique comprend au moins deux aimants à aimantation diamétrale disposés axialement dans le prolongement l'un de l'autre en étant espacés l'un de l'autre, de sorte que le pôle de l'un des aimants soit situé en regard d'un pôle opposé de l'autre aimant, et au moins deux électrodes disposées respectivement autour des aimants.

Dans divers modes de réalisation, au moins l'une des électrodes est fixée par vissage sur le corps.

Dans un autre mode de réalisation, l'électrode entoure l'aimant et comporte un ensemble de lumières comprenant chacune deux zones en regard électriquement conductrices et délimitant chacune un entrefer.

L'aimant peut être électriquement conducteur et constituer l'une des électrodes de la tête magnétique.

Dans un autre mode de réalisation, les électrodes comportent chacune un ensemble de dents s'étendant axialement à partir d'un support, les électrodes étant coaxialement placées l'une dans l'autre et autour de l'aimant, de sorte que les dents de l'une des électrodes soient placées entre les dents de l'autre électrode avec interposition d'un isolant.

Dans divers modes de réalisation, les électrodes peuvent chacune être réalisées en matériau électriquement conducteur revêtu d'un matériau isolant et comprendre des zones électriquement conductrices dépourvues d'isolant à l'endroit de la zone d'entrefer et des moyens de raccordement électrique des électrodes.

L'isolant est par exemple formé par surmoulage d'un matériau isolant.

Dans divers modes de réalisation, la tête magnétique comporte avantageusement des moyens de fixation de la tête magnétique sur un réceptacle et des moyens de verrouillage des moyens de fixation.

Par exemple, les moyens de fixation comprennent des moyens de fixation à baïonnette, les moyens de verrouillage comprenant une bague de verrouillage apte à empêcher une rotation des moyens de fixation à baïonnette.

Avantageusement, l'aimant est un aimant permanent en Néodyme Fer Bore (NdFeB).

L'invention a également pour objet un détecteur magnétique de particules métalliques dans un circuit hydraulique comprenant une tête magnétique telle que définie ci-dessus et un réceptacle dans lequel vient se loger la tête magnétique.

Dans un mode de réalisation, le réceptacle comprend un capot d'extrémité dans lequel vient se loger l'aimant.

Par exemple, le capot d'extrémité du réceptacle constitue l'une des électrodes du détecteur magnétique et comporte un ensemble de lumières délimitant chacune un entrefer, chaque lumière comportant deux zones en regard électriquement conductrices.

Avantageusement, le capot d'extrémité comporte un clapet sollicité à l'état fermé et actionnable à l'ouverture sous l'action de la tête magnétique.

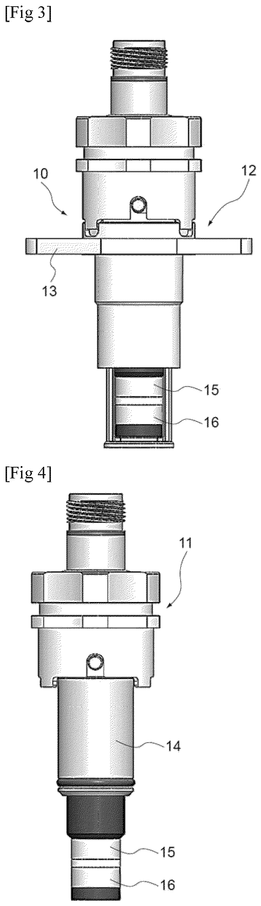

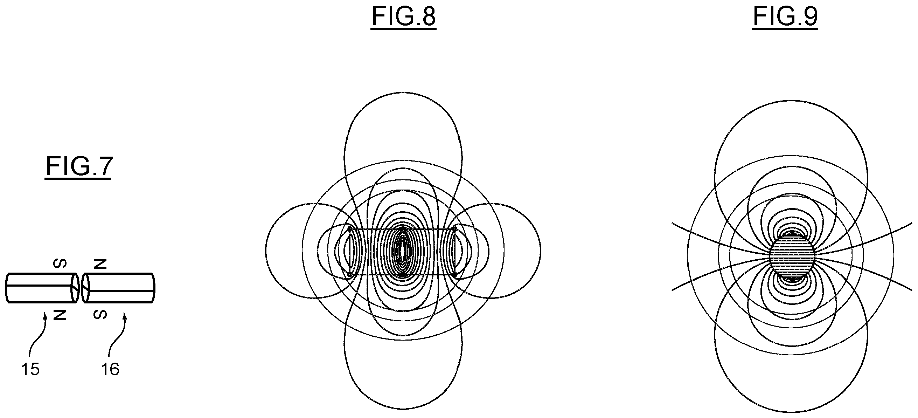

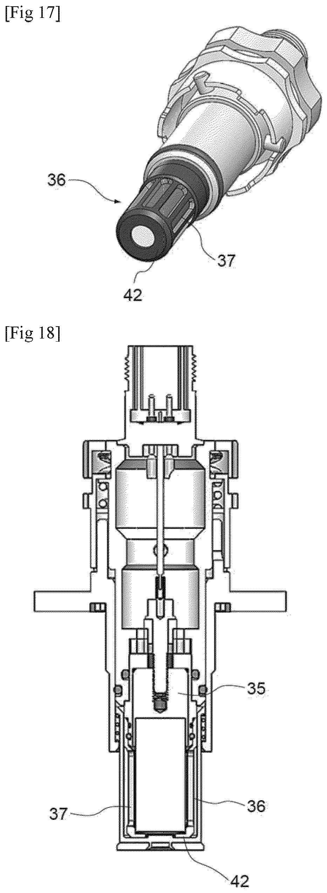

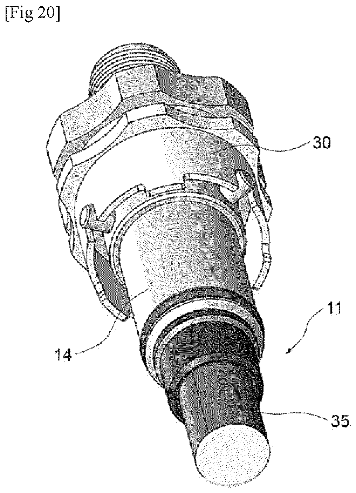

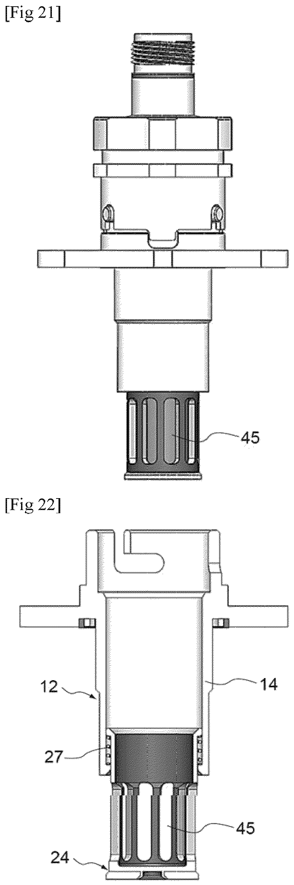

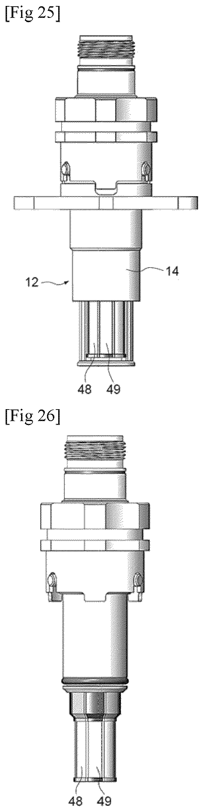

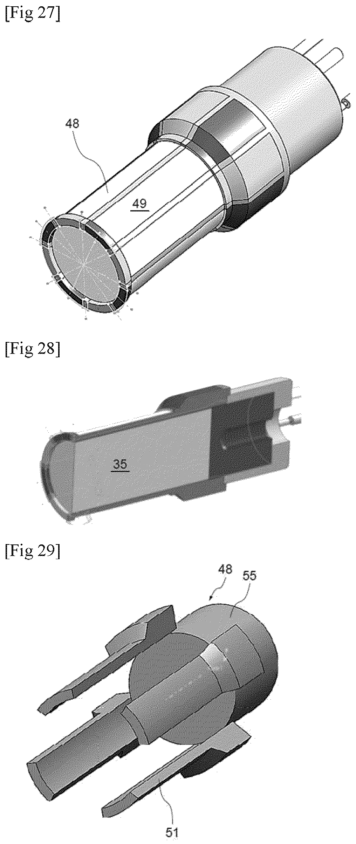

D'autres buts, caractéristiques et avantages de l'invention apparaîtront à la lecture de la description suivante, donnée uniquement à titre d'exemple et faite en référence aux dessins annexés sur lesquels:
[Fig 1]
[Fig 2]
   dont il a déjà été fait mention, illustrent deux modes de réalisation d'un détecteur magnétique selon l'état de la technique, respectivement à entrefer axial et à entrefer radial ;
[Fig 3]
   illustre un premier mode de réalisation d'un détecteur magnétique selon l'invention ;
[Fig 4]
[Fig 5]
   illustrent respectivement une tête magnétique et un réceptacle du détecteur de la figure 3 ;
[Fig 6]
   est une vue en coupe du détecteur magnétique de la figure 3 ;
[Fig 7]
   illustre un exemple de réalisation d'un aimant à aimantation diamétrale du détecteur magnétique de la figure 3 ;
[Fig 8]
[Fig 9]
   sont des vues respectivement latérale et en coupe de l'aimant de la figure 7, montrant les lignes de champ magnétique ;
[Fig 10]
[Fig 11]
[Fig 12]
[Fig 13]
[Fig 14]
   illustrent les moyens de fixation de la tête magnétique sur le réceptacle et les moyens de verrouillage des moyens de fixation ;
[Fig 15]
   montre un détail du moyen de verrouillage ;
[Fig 16a]
   illustre un exemple de réalisation des électrodes ;
[Fig 16b]
   illustre un exemple de réalisation d'isolant entre électrodes ;
[Fig 17]
   illustre un autre mode de réalisation d'une tête magnétique selon un deuxième mode de réalisation ;
[Fig 18]
   est une vue en coupe de la tête magnétique de la figure 17 ;
[Fig 19]
   est une vue en coupe à plus grande échelle du détecteur magnétique de la figure 17, montrant les chemins de continuité électrique ;
[Fig 20]
   illustre un autre mode de réalisation d'une tête magnétique conforme à l'invention ;
[Fig 21]
   est une vue latérale d'un détecteur magnétique doté d'une tête magnétique selon la figure 20
[Fig 22]
   est une vue en coupe du réceptacle du détecteur magnétique de la figure 21 ;
[Fig 23]
   est une vue en coupe du détecteur de la figure 21 montrant les chemins de continuité électrique dans la tête magnétique et dans le réceptacle ;
[Fig 24]
   est une vue en perspective du détecteur magnétique de la figure 21 en présence d'une particule métallique ;
[Fig 25]
   est une vue latérale d'un détecteur magnétique selon encore un autre mode de réalisation ;
[Fig 26]
   illustre la tête magnétique du détecteur de la figure 25 ;
[Fig 27]
   est une vue de détail de l'extrémité de la tête magnétique de la figure 26, montrant la structure de l'aimant et des électrodes ;
[Fig 28]
   est une vue en coupe axiale de la tête magnétique de la figure 27 ;
[Fig 29]
[Fig 30]
   montrent la structure des électrodes de la tête magnétique de la figure 27 ; et

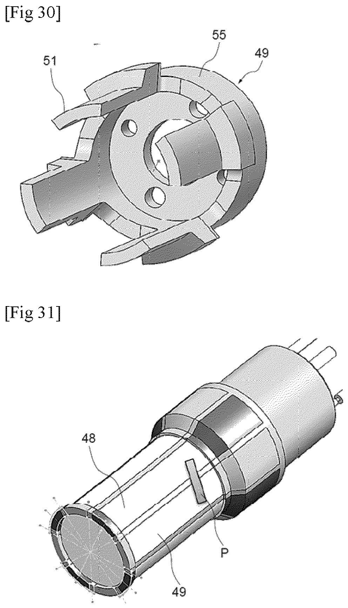

[Fig 31] illustre l'extrémité de la tête magnétique de la figure 27 en présence d'une particule métallique P.

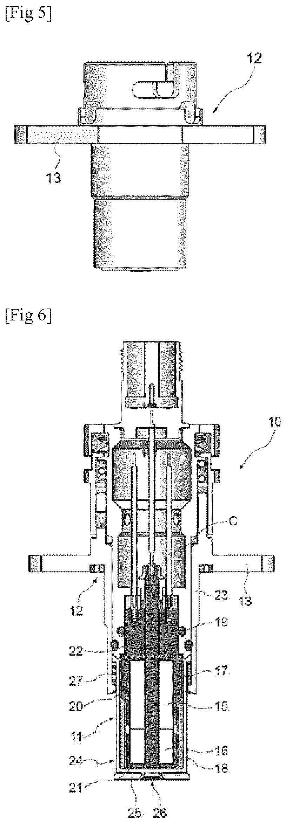

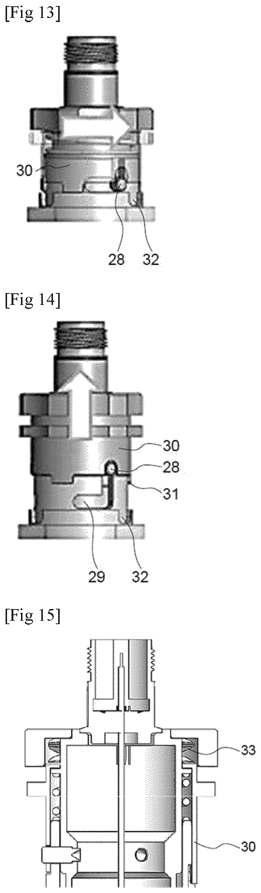

On se réfère tout d'abord aux figures 3 à 15 et 16a et 16b qui illustrent un premier mode de réalisation d'un détecteur magnétique de particules métalliques et d'une tête magnétique correspondante selon premier mode de réalisation.

Dans le mode de réalisation envisagé, le détecteur magnétique est destiné à être monté dans un carter d'huile d'un système de motorisation d'un aéronef. L'invention n'est toutefois pas limitée à cette application et couvre, de manière générale, la détection de particules métalliques dans un liquide dans un circuit hydraulique.

En se référant tout d'abord aux figures 3 à 5, le détecteur magnétique, désigné par la référence numérique générale 10, comprend essentiellement une tête magnétique 11 qui vient se monter dans un réceptacle 12. Tel qu'illustré, l'ensemble vient se monter sur le carter au moyen d'une collerette 13 prévue sur le réceptacle qui coopère avec un système vis-écrou prévu sur le carter. Bien entendu, d'autres moyens de fixation, par exemple au moyen de goujons, peuvent également être utilisés en variante. Le détecteur magnétique peut également être monté sur le carter au moyen d'un filetage prévu directement sur la surface périphérique externe du réceptacle.

La tête magnétique 11 comprend un corps axial 14 de forme générale cylindrique dans lequel vient se monter un ou plusieurs aimants 15 et 16 à aimantation diamétrale et une ou plusieurs électrodes 17 et 18 correspondantes. Dans le mode de réalisation illustré sur les figures 1 à 15 et 16a et 16b, la tête magnétique comporte une électrode proximale 17 qui s'étend essentiellement dans le corps 14 et une électrode distale 18.

En référence aux figures 7 à 9, la tête magnétique comprend deux aimants cylindriques aimantés diamétralement, mis bout à bout, dans le prolongement l'un de l'autre en étant espacés axialement et de sorte que le pôle N de l'un des aimants soit situé en regard du pôle S opposé de l'autre aimant.

Cette disposition des aimants à aimantation diamétrale permet de créer dans l'entrefer entre les deux aimants un champ magnétique dont les lignes de champ tendent à aligner les particules, ce qui est favorable pour améliorer la détection. L'orientation diamétrale de l'aimantation permet d'optimiser la captation et la mise en opposition des deux aimants permet d'améliorer la détection des particules dans l'entrefer entre les deux aimants.

Les aimants ont en réalité une section en coupe transversale annulaire et sont maintenus entre les électrodes 17 et 18. La première électrode proximale 17 comporte une base 19 fixée par vissage au corps de la tête magnétique et une jupe cylindrique 20 entourant le premier aimant 15.

La deuxième électrode distale 18 comporte également une embase 21 et un prolongement cylindrique s'étendant à partir de l'embase. Elle comporte une tige axiale 22 pour sa fixation par vissage sur le corps de la tête magnétique.

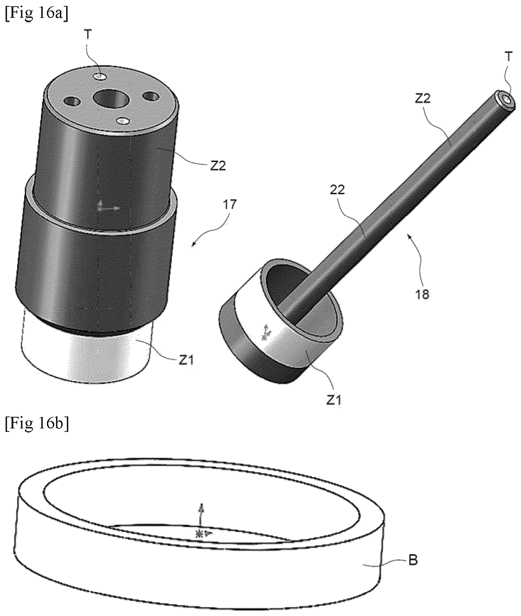

L'extrémité proximale de chaque électrode est dotée de moyens de raccordement électrique au calculateur de l'aéronef. Ces moyens de raccordement électrique peuvent être constitués, tel qu'illustré, par des cosses C qui s'insèrent dans des trous taraudés T (figure 16a) pratiqués dans la tige axiale 22 et dans l'électrode proximale 17.

Le réceptacle 12 comporte un corps 23 cylindrique, dans lequel vient se loger la tête magnétique, et un capot d'extrémité 24 comprenant un clapet 25, fixé par exemple sur le capot d'extrémité 24 par un sertissage 26, normalement fermé, mais susceptible d'être ouvert sous l'action de la tête magnétique lorsqu'elle est entièrement insérée dans le réceptacle, un ressort 27 étant interposé entre le capot d'extrémité 24 et le réceptacle.

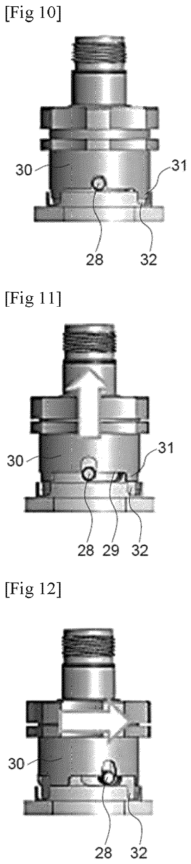

En référence aux figures 10 à 15, le détecteur est par ailleurs doté de moyens de fixation de la tête de détection dans le réceptacle et de moyens de verrouillage des moyens de fixation.

Les moyens de fixation de la tête magnétique dans le réceptacle comportent des moyens de fixation à baïonnette. La tête magnétique comprend à cet égard au moins deux pions 28 radiaux s'engageant dans au moins deux encoches 29 respectives en forme de L pratiquées dans le réceptacle.

Les moyens de verrouillage sont quant à eux formés par une bague de verrouillage 30 montée à rotation sur la tête magnétique et qui comporte au moins deux saillies 31 qui s'engagent dans au moins deux encoches correspondantes 32 pratiquées dans le réceptacle et dans la bague.

Un ressort 33 sollicite la bague de verrouillage pour maintenir les saillies 31 dans les encoches 32 empêchant, de la sorte, la rotation de la tête magnétique par rapport au réceptacle.

Ainsi, pour déverrouiller la tête, et éviter de la sorte toute déconnexion intempestive de la tête magnétique, il convient en premier lieu de dégager la bague de verrouillage 30 en la tirant vers le haut à l'encontre de l'effort exercé par le ressort 33. La tête magnétique peut alors être tournée jusqu'à ce qu'elle puisse être dégagée du réceptacle.

Un tel agencement permet également d'éviter tout montage incorrect de la tête magnétique dans le réceptacle, le replacement de la bague de verrouillage sous l'action du ressort 33 permettant d'indiquer aux opérateurs que la tête magnétique est correctement montée sur le réceptacle.

En référence aux figures 16a et 16b sur lesquelles les zones relativement claires Z1 représentent les zones conductrices et sur lesquelles les zones Z2 relativement sombres sont des zones isolantes, comme indiqué précédemment, la tête magnétique comprend deux électrodes 17 et 18 coaxiales. Ces électrodes sont réalisées en matériau électriquement conducteur revêtu d'un isolant. Des zones électriquement conductrices Z1 sont ménagées notamment en regard de la zone d'entrefer.

Par exemple, les électrodes sont réalisées en aluminium et sont revêtues d'une couche d'isolant formée par traitement d'anodisation sulfurique dure de manière à former, par exemple, une couche de revêtement isolant d'épaisseur comprise entre 30 et 50 microns, certaines zones des électrodes étant par la suite réusinées (ou épargnées lors du revêtement) puis traitées par conversion chimique, de type alodine, pour garantir la continuité électrique.

Bien entendu, on ne sort pas du cadre de l'invention lorsque les électrodes sont réalisées en un autre matériau ou sont revêtues d'une couche d'isolant ayant une autre nature.

On peut, par exemple, en variante, revêtir les électrodes d'une couche de matériau isolant et pratiquer les zones électriquement conductrices par usinage local, ou encore revêtir localement les électrodes d'une couche de vernis avant application du matériau isolant, ces couches de vernis étant par la suite supprimées pour laisser à nu les zones devant être électriquement conductrices.

Les zones électriquement conductrices Z1 des électrodes sont réalisées dans la zone d'entrefer et dans la zone de raccordement électrique des électrodes, à l'endroit des vis de fixation.

Comme le montre la figure 16b, l'entrefer est ici constitué par une bague B isolante, par exemple en matière plastique.

Dans le mode de réalisation qui vient d'être décrit, la tête magnétique comporte deux aimants à aimantation diamétrale placés dans le prolongement l'un de l'autre.

Il est également possible, en variante, comme visible sur les figures 17 à 20, de doter la tête magnétique d'un seul aimant cylindrique qui constitue, en outre, l'une des électrodes de la tête magnétique.

Dans ce cas, l'aimant, désigné par la référence 35, est un aimant électriquement conducteur. Il est par exemple revêtu d'une couche de nickel.

La deuxième électrode 36 est formée par une pièce cylindrique rapportée qui entoure l'aimant et qui comporte un ensemble de lumières 37 délimitées par un matériau électriquement conducteur. Ces lumières comportent des bords périphériques et en particulier des faces périphériques en regard électriquement conductrices.

Dans ce mode de réalisation, la deuxième électrode 36 peut être maintenue sur la tête magnétique au moyen d'un joint 39.

Ainsi, seul l'aimant, qui constitue l'une des électrodes, est fixé par vissage sur le corps de la tête magnétique.

Bien que le mode de réalisation décrit précédemment en référence aux figures 3 à 15 soit avantageux dans la mesure où l'aimant est protégé par un fourreau formé par les 2 électrodes dans le prolongement l'une de l'autre, ce mode de réalisation est avantageux dans la mesure où il permet d'avoir un aimant de volume accru, grâce à la suppression de l'une des électrodes.

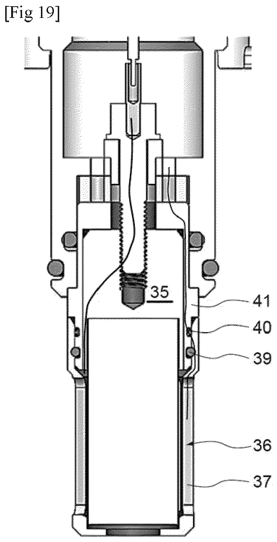

En référence à la figure 19, la continuité électrique au sein de la tête magnétique est obtenue par la vis centrale qui assure la fixation de l'aimant, par l'aimant lui-même électriquement conducteur et, de l'autre côté des entrefers, par la deuxième électrode 36, par un ressort de continuité électrique 40 et une pièce d'assemblage cylindrique 41 électriquement conductrice interposée entre l'aimant et le corps de la tête magnétique.

Ce circuit électrique est fermé ou, en tout état de cause, sa résistance est réduite lorsqu'une particule métallique est présente dans l'entrefer entre l'aimant et la deuxième électrode, de sorte qu'un courant électrique peut circuler depuis la zone conductrice jusqu'aux fils électriques de la tête magnétique.

Dans ce mode de réalisation, comme indiqué précédemment, l'aimant présente un volume important. Ce mode de réalisation présente en outre une architecture simple, dont l'assemblage est facile à réaliser.

En outre, la deuxième électrode 36 comporte plusieurs entrefers 37, localisés chacun entre l'aimant et une lumière 37, ce qui augmente le nombre de zones de détection des particules métalliques.

Bien que dans les modes de réalisations décrits la tête magnétique ne comporte qu'un seul élément, il est également possible, en variante, d'utiliser plusieurs aimants.

On notera enfin, comme le montre la figure 18, que la deuxième électrode 36 comporte, dans sa zone d'extrémité, un bord annulaire 42 électriquement isolant recouvrant partiellement l'extrémité de l'aimant de sorte que, en l'absence de l'électrode 36 amovible, par exemple suite à une omission lors d'un remontage, une continuité électrique apparaît entre l'aimant et le réceptacle, signalant le défaut d'assemblage.

Dans le deuxième mode de réalisation décrit précédemment en référence aux figures 17 à 22, la tête magnétique comporte deux électrodes, constituées l'une par l'aimant et l'autre par une électrode rapportée 36 montée sur le corps de la tête magnétique par l'intermédiaire d'une pièce de maintien, en l'espèce le joint 39.

Dans un autre mode de réalisation visible sur les figures 20 à 23, sur laquelle on reconnaît la tête magnétique 11 dotée de son corps axial 14 de forme générale cylindrique et équipée des moyens de fixation à baïonnette et de la bague de verrouillage 30, et sur laquelle on reconnaît également l'aimant 35 à magnétisation diamétrale qui constitue l'une des électrodes de la tête magnétique, dans ce mode de réalisation, la deuxième électrode est formée dans le réceptacle 12.

Le capot d'extrémité 24 du réceptacle, qui est monté de manière coulissante par rapport au corps 14 avec interposition d'un ressort 27, est ici doté d'un ensemble de lumières, tels que 45, dont les bords longitudinaux sont électriquement conducteurs, par exemple par usinage réalisé après le dépôt d'un revêtement isolant.

Ce mode de réalisation est avantageux dans la mesure où la deuxième électrode est remplacée par le capot d'extrémité 24 du réceptacle et permet ainsi d'augmenter le volume de l'aimant.

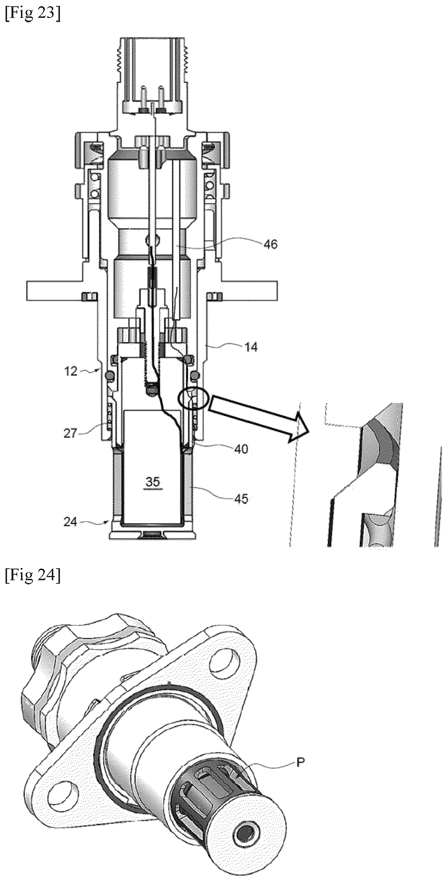

Comme le montre la figure 23, dans ce mode de réalisation, le chemin de continuité électrique passe, d'une part axialement, dans le corps du réceptacle et le long du revêtement conducteur de l'aimant et, d'autre part, le long du capot 24, de la pièce 40 d'assemblage cylindrique puis vers une cosse 46 interne.

Ce circuit de continuité électrique se ferme ou sa résistance électrique diminue lorsqu'une particule P de métal se loge entre l'aimant et le capot d'extrémité de la tête magnétique.

Comme dans le mode de réalisation décrit précédemment, ce mode de réalisation permet d'augmenter le diamètre de l'aimant et, par conséquent, son volume.

Les parties essentielles de la tête magnétique présentent une forme cylindrique, ce qui facilite le nettoyage des particules lors des opérations de maintenance, en vue de leur analyse.

Ce mode de réalisation présente également plusieurs entrefers, ce qui augmente le nombre de zones de détection.

Dans ce mode de réalisation, la tête magnétique comporte un seul aimant. Il est également possible, en variante, d'utiliser deux aimants cylindriques soudés pour concentrer les particules.

Les figures 25 à 31 illustrent un quatrième mode de réalisation d'une tête magnétique et d'un détecteur magnétique.

Ce mode de réalisation diffère du mode de réalisation décrit précédemment en référence aux figures 20 à 24 en ce que la tête magnétique comprend plusieurs aimants à aimantation diamétrale.

Les électrodes, telles que 48 et 49, sont réparties angulaires autour des aimants, avec interposition d'un revêtement isolant, par exemple par surmoulage d'élastomère, de plastique ou de résine, ce surmoulage s'étendant entre les électrodes et les aimants, d'une part, et entre les électrodes, d'autre part.

Les électrodes 48 et 49 sont réalisées chacune en une pièce qui comporte, en ce qui concerne l'électrode 48 et l'électrode 49, un support 55 à partir duquel s'étend un ensemble de dents 51. Tel que représenté sur les figures 29 et 30, l'électrode 48 vient se monter dans l'autre électrode 49 de manière coaxiale de sorte que les dents de l'une des électrodes soient intercalées chacune entre deux dents de l'autre électrode, l'ensemble étant par la suite surmoulé.

Dans ce mode de réalisation, comme illustré à la figure 31, le circuit de continuité électrique se ferme lorsqu'une particule magnétique P s'étend entre deux électrodes consécutives 48 et 49.

Comme décrit précédemment, chaque électrode est par exemple réalisée en aluminium revêtu d'un revêtement faisant office de protection contre la corrosion et assurant une isolation électrique. Il s'agit par exemple d'une oxydation anodique sulfurique dure. Certaines parties des électrodes sont usinées afin de supprimer le revêtement et de mettre à nu l'aluminium. Les différents éléments sont assemblés. Puis un surmoulage d'élastomère est réalisé.

Bien entendu, on ne sort pas de du cadre dans l'invention lorsque d'autres processus sont utilisés, tels qu'un enrobage de résine. Un usinage peut ensuite être réalisé de façon à supprimer localement le revêtement isolant et obtenir une zone électriquement conductrice sur chaque électrode.

Dans les divers modes de réalisation qui ont été décrits, les aimants sont avantageusement des aimants en Neodyme. On pourra par exemple envisager des grades Neodyme Fer Bore, de type 45SH, qui présente des puissances importantes tout en résistant à des températures élevées, par exemple, de l'ordre de 150°C.

## Revendications

1. Tête magnétique (11) pour détecteur magnétique de particules métalliques dans un circuit hydraulique, comprenant un corps axial (14) comportant intérieurement au moins un aimant (15, 16), au moins une première électrode (17, 18) délimitant une zone d'entrefer (E) située dans le champ magnétique créé par l'aimant, de sorte que le circuit crée dans l'entrefer une zone d'alignement de particules et des moyens (C) de raccordement électrique des électrodes, **caractérisé en ce que** l'aimant (15, 16 ; 35) est un aimant à aimantation diamétrale.

2. Tête magnétique selon la revendication 1, comprenant au moins deux aimants (15, 16) à aimantation diamétrale disposés axialement dans le prolongement l'un de l'autre en étant espacés l'un de l'autre, de sorte que le pôle de l'un des aimants soit situé en regard d'un pôle opposé de l'autre aimant, et au moins deux électrodes respectivement disposées autour des aimants.

3. Tête magnétique selon revendication 2, dans laquelle au moins l'une des électrodes est fixée par visage sur le corps.

4. Tête magnétique selon l'une revendication 1, dans laquelle l'électrode comporte un ensemble de lumières (37) comprenant chacune deux zones en regard électriquement conductrices et délimitant chacune un entrefer.

5. Tête magnétique selon la revendication 4, dans laquelle l'aimant est électriquement conducteur et constitue l'une des électrodes de la tête magnétique.

6. Tête magnétique selon la revendication 1, dans laquelle les électrodes comportent chacune un ensemble de dents (51) s'étendant axialement à partir d'un support (55), les électrodes étant coaxialement placées l'une dans l'autre autour de l'aimant, de sorte que les dents de l'une des électrodes soient placées entre les dents de l'autre électrode avec interposition d'un isolant.

7. Tête magnétique selon l'une quelconque des revendications 1 à 6, dans laquelle les électrodes sont chacune réalisées en un matériau électriquement conducteur revêtu d'un matériau isolant et comportent des zones électriquement conductrices dépourvues d'isolant à l'endroit de la zone d'entrefer (E) et des moyens (C) de raccordement électrique des électrodes.

8. Tête magnétique selon revendication 7, dans laquelle l'isolant est formé par surmoulage d'un matériau isolant.

9. Tête magnétique selon l'une quelconque des revendications 1 à 8, comprenant des moyens (28, 29) de fixation de la tête magnétique sur un réceptacle et des moyens (30, 31) de verrouillage des moyens de fixation.

10. Tête magnétique selon la revendication 9, dans laquelle les moyens de fixation (28, 29) comprennent des moyens de fixation à baïonnette, les moyens de verrouillage (30, 31) comprenant une bague de verrouillage apte à empêcher une rotation des moyens de fixation à baïonnette.

11. Tête magnétique selon l'une quelconque des revendications 1 à 10, dans laquelle l'aimant est un aimant permanent en Neodyme.

12. Détecteur magnétique de particules métalliques dans un circuit hydraulique, comprenant une tête magnétique (11) selon l'une quelconque des revendications 1 à 11 et un réceptacle (12) dans lequel vient se loger la tête magnétique.

13. Détecteur magnétique selon la revendication 12, dans lequel le réceptacle comprend un capot d'extrémité (24) dans lequel vient se loger l'aimant.

14. Détecteur magnétique selon la revendication 13, dans lequel le capot d'extrémité (24) du réceptacle constitue l'une des électrodes du détecteur magnétique et comporte un ensemble de lumières (45) délimitant chacune un entrefer, chaque lumière comprenant deux zones en regard électriquement conductrices.

15. Détecteur magnétique selon la revendication 13, dans lequel le capot d'extrémité comporte un clapet (25) sollicité à l'état fermé et actionnable à l'état ouvert sous l'action de la tête magnétique (11).

## Patentansprüche

1. Magnetkopf (11) für einen Magnetdetektor zum Nachweis von Metallteilchen in einem Hydraulikkreis, der einen axialen Körper (14), der innen mindestens einen Magnet (15, 16) aufweist, mindestens eine erste Elektrode (17, 18), die eine Spaltzone (E) begrenzt, die sich in dem vom Magnet erzeugten Magnetfeld befindet, so dass der Kreis im Spalt eine Teilchenausrichtzone erzeugt und elektrische Anschlussmittel (C) der Elektroden umfasst, **dadurch gekennzeichnet, dass** der (15, 16; 35) ein Magnet mit diametraler Magnetisierung ist.

2. Magnetkopf nach Anspruch 1, der mindestens zwei Magnete (15, 16) mit diametraler Magnetisierung, die axial in der Verlängerung zueinander angeordnet und beabstandet sind, so dass sich der Pol von einem der Magnete einem entgegengesetzten Pol des anderen Magnets zugewandt befindet, und mindestens zwei Elektroden, die jeweils um Magnete angeordnet sind, umfasst.

3. Magnetkopf nach Anspruch 2, wobei mindestens eine der Elektroden durch Schrauben auf dem Körper befestigt ist.

4. Magnetkopf nach einem Anspruch 1, wobei die Elektrode eine Anordnung von Öffnungen (37) aufweist, die jeweils zwei zugewandte elektrisch leitende und jeweils einen Spalt begrenzende Zonen umfassen.

5. Magnetkopf nach Anspruch 4, wobei der Magnet elektrisch leitend ist und eine der Elektroden des Magnetkopfs darstellt.

6. Magnetkopf nach Anspruch 1, wobei die Elektroden jeweils eine Anordnung von Zähnen (51) aufweisen, die sich axial ab einem Halter (55) erstrecken, wobei die Elektroden koaxial ineinander um den Magnet derart platziert sind, dass die Zähne der einen von den Elektroden zwischen den Zähnen der anderen Elektrode mit Zwischenstellung einer Isolation platziert sind.

7. Magnetkopf nach einem der Ansprüche 1 bis 6, wobei jede von den Elektroden aus einem elektrisch leitenden Material hergestellt ist, das mit einem Isolationsmaterial beschichtet ist und elektrisch leitende Zonen ohne Isolation an der Stelle der Spaltzone (E) und elektrische Anschlussmittel (C) der Elektroden aufweist.

8. Magnetkopf nach Anspruch 7, wobei die Isolation durch Aufformen eines Isolationsmaterials gebildet ist.

9. Magnetkopf nach einem der Ansprüche 1 bis 8, der Befestigungsmittel (28, 29) des Magnetkopfs auf einem Behälter und Verriegelungsmittel (30, 31) der Befestigungsmittel umfasst.

10. Magnetkopf nach Anspruch 9, wobei die Befestigungsmittel (28, 29) Bajonettbefestigungsmittel umfassen, wobei die Verriegelungsmittel (30, 31) einen Verriegelungsring umfassen, der imstande ist, eine Rotation der Bajonettbefestigungsmittel zu verhindern.

11. Magnetkopf nach einem der Ansprüche 1 bis 10, wobei der Magnet ein Dauermagnet aus Neodym ist.

12. Magnetdetektor zum Nachweis von Metallteilchen in einem Hydraulikkreis, der einen Magnetkopf (11) nach einem der Ansprüche 1 bis 11 und einen Behälter (12), in dem der Magnetkopf untergebracht ist, umfasst.

13. Magnetdetektor nach Anspruch 12, wobei der Behälter eine Endabdeckung (24) umfasst, in der der Magnet untergebracht ist.

14. Magnetdetektor nach Anspruch 13, wobei die Endabdeckung (24) des Behälters eine der Elektroden des Magnetdetektors darstellt und eine Anordnung von Öffnungen (45) aufweist, von denen jede einen Spalt begrenzt, wobei jede Öffnung zwei elektrisch leitende einander zugewandte Zonen umfasst.

15. Magnetdetektor nach Anspruch 13, wobei die Endabdeckung ein unter der Wirkung des Magnetkopfs (11) im geschlossenen Zustand befindliches und in den geöffneten Zustand betätigbares Ventil (25) aufweist.

## Claims

1. A magnetic head (11) for a magnetic detector for detecting metal particles in a hydraulic circuit comprising an axial body (14) internally comprising at least one magnet (15, 16), at least a first electrode (17, 18) defining an air gap (E) zone located in the magnetic field created by the magnet, such that the circuit creates a particle alignment zone in the air gap, and means (C) for electrically connecting the electrodes, **characterized in that** the magnet (15, 16; 35) is a diametrically magnetized magnet.

2. The magnetic head according to claim 1, comprising at least two diametrically magnetized magnets (15, 16) arranged axially, one as an extension of the other and spaced apart from each other, so that the pole of one of the magnets is located in front of an opposite pole of the other magnet, and at least two electrodes arranged around the magnets, respectively.

3. The magnetic head according to claim 2, wherein at least one of the electrodes is secured to the body by threaded fastener.

4. The magnetic head according to claim 1, wherein the electrode includes a set of openings (37) each of which comprising two electrically conductive zones that are opposite each other and each one defining an air gap.

5. The magnetic head according to claim 4, wherein the magnet is electrically conductive and constitutes one of the electrodes of the magnetic head.

6. The magnetic head according to claim 1, wherein each electrode includes a set of teeth (51) extending axially from a base (55), the electrodes being placed coaxially one inside the other around the magnet in such a way that the teeth of one of the electrodes are placed between the teeth of the other electrode with an insulator placed in between.

7. The magnetic head according to one of claims 1 to 6, wherein each electrode is made of an electrically conductive material coated with an insulating material, and including electrically conductive zones without insulation in the location of the air gap zone (E), and means (C) for electrically connecting the electrodes.

8. The magnetic head according to claim 7, wherein the insulator is provided by overmoulding with an insulating material.

9. The magnetic head according to one of claims 1 to 8, comprising means (28, 29) for attaching the magnetic head to a receptacle and means (30, 31) for locking the attachment means.

10. The magnetic head according to claim 9, wherein the attachment means (28, 29) comprise twist-lock attachment means, the locking means (30, 31) comprising a lock ring capable of preventing a rotation of the twist-lock attachment means.

11. A magnetic head according to one of claims 1 to 10, wherein the magnet is a permanent magnet made of Neodymium.

12. A magnetic metal particle detector in a hydraulic circuit, said detector comprising a magnetic head (11) according to one of claims 1 to 11 and a receptacle (12) in which the magnetic head is placed.

13. The magnetic detector according to claim 12, wherein the receptacle comprises an end cap (24) in which the magnet is housed.

14. The magnetic detector according to claim 13, wherein the end cap (24) of the receptacle constitutes one of the electrodes of the magnetic detector and includes a set of openings (45) each of which defining an air gap, each opening comprising two electrically conductive zones that are opposite each other.

15. The magnetic detector according to claim 13, wherein the end cap includes a flap (25) drawn to the closed state and which can be actuated to the open state by the action of the magnetic head (11).
